**Europäisches Patentamt**

**European Patent Office**

**Office européen des brevets**

(19)

(11) Veröffentlichungsnummer : **0 208 933**
**B1**

(12)

# EUROPÄISCHE PATENTSCHRIFT

(45) Veröffentlichungstag der Patentschrift :
**17.05.89**

(21) Anmeldenummer : **86108228.7**

(22) Anmeldetag : **16.06.86**

(51) Int. Cl.⁴ : **B 01 J 23/64**, B 01 J 27/02,
C 07 C 37/07// C07C37/06

(54) **Trägerkatalysator, Verfahren zu seiner Herstellung sowie dessen Verwendung zur Herstellung von Hydroxydiphenyl.**

(30) Priorität : **28.06.85 DE 3523205**

(43) Veröffentlichungstag der Anmeldung :
**21.01.87 Patentblatt 87/04**

(45) Bekanntmachung des Hinweises auf die Patenterteilung : **17.05.89 Patentblatt 89/20**

(84) Benannte Vertragsstaaten :
**CH DE FR GB IT LI**

(56) Entgegenhaltungen :
**EP—A— 0 001 425**
**EP—A— 0 018 763 .**
**FR—A— 2 177 951**
**US—A— 4 288 347**

(73) Patentinhaber : **BAYER AG**
**Konzernverwaltung RP Patentabteilung**
**D-5090 Leverkusen 1 Bayerwerk (DE)**

(72) Erfinder : **Immel, Otto, Dr.**
**Immenhofweg 26**
**D-4150 Krefeld (DE)**
Erfinder : **Weissel, Oskar, Dr.**
**Deswatinesstrasse 70**
**D-4150 Krefeld (DE)**
Erfinder : **Schwarz, Hans-Helmut, Dr.**
**Ratherstrasse 90**
**D-4150 Krefeld (DE)**

EP 0 208 933 B1

**EP 0 208 933 B1**

## Beschreibung

Die Erfindung betrifft einen neuen Trägerkatalysator sowie ein Verfahren zu seiner Herstellung und seine Verwendung zur Herstellung von Hydroxydiphenyl.

Aus der DE-A-22 11 721 ist ein Verfahren zur Herstellung von o-Phenylphenol (2-Hydroxydiphenyl) durch Dehydrierung von Cyclohexanon-Derivaten in Gegenwart eines Trägerkatalysators bekannt. Der bei diesem Verfahren eingesetzte Katalysator wird hergestellt, indem eine sehr kleine Menge von Palladium, Platin, Iridium oder Rhodium oder ein Gemisch von zwei oder mehreren dieser Elemente auf einen Träger wie Kieselerde, Aluminiumoxid, Kieselerde-Aluminiumoxid oder Aktivkohle abgeschieden wird und in dem weiterhin eine geeignete Menge eines Alkalis hinzugegeben wird. In der genannten Offenlegungsschrift wird besonders herausgestellt, daß Platin und Palladium eine besonders hohe Wirksamkeit für die selektive Bildung des gewünschten Produktes aufweisen. Die Edelmetallkatalysatoren, die bei diesem Verfahren eingesetzt werden, besitzen jedoch entweder eine zu geringe Selektivität (insbesondere bei Einsatz eines rhodiumhaltigen Katalysators), eine unzureichende Aktivität oder eine zu kurze Lebensdauer (vgl. in diesem Zusammenhang das Vergleichsbeispiel).

Weiterhin ist aus der deutschen Patentschrift 20 49 809 bekannt, Hydroxydiphenyl durch katalytische Dehydrierung von Verbindungen und/oder Verbindungsgemischen, die aus ganz und/oder teilweise hydriertem Hydroxydiphenyl bestehen, herzustellen in Gegenwart eines Nickel, Chrom, Aluminium, Kupfer und Alkali enthaltenden Dehydrierungskatalysators. In der US-A-40 60 559 wird ebenfalls ein Verfahren zur Herstellung von o-Phenylphenol durch Dehydrierung von Cyclohexylphenol beschrieben, bei dem in Gegenwart eines platin- oder palladiumhaltigen Trägerkatalysators gearbeitet wird. Nachteilig bei diesen Verfahren sind ebenfalls die relativ kurze Lebensdauer der Katalysatoren und die zum Teil unzureichende Aktivität.

Es wurde nun ein neuer Trägerkatalysator gefunden, der dadurch gekennzeichnet ist, daß er einen Gehalt an Rhodium von 0,1 bis 5 Gew.-%, einen Gehalt an Chrom und Mangan von zusammen 0,05 bis 8 Gew.-%, einen Gehalt an Alkalimetall von 0,05 bis 15 Gew.-% und gegebenenfalls einen Schwefelgehalt von 0,05 bis 3 Gew.-%, bezogen auf das Trägermaterial, aufweist.

Als Trägermaterial kommen die üblichen Katalysatorträger in Frage, insbesondere $\alpha$- und $\gamma$-Aluminiumoxid, Kieselgel, Kieselgur, Montmorillonite, Bimsstein und/oder Aktivkohle, vorzugsweise $\gamma$-Aluminiumoxid mit einer hohen spezifischen Oberfläche ($> 50$ m², bevorzugt 150 bis 400 m²/g).

Der erfindungsgemäße Trägerkatalysator weist bevorzugt einen Gehalt an Rhodium von 0,2 bis 2 Gew.-%, einen bevorzugten Gehalt an Chrom und Mangan von zusammen 0,2 bis 5 Gew.-%, einen bevorzugten Gehalt an Alkalimetall von 0,1 bis 10 Gew.-%, und gegebenenfalls einen bevorzugten Schwefelgehalt von 0,1 bis 1,6 Gew.-%, bezogen auf das Trägermaterial, auf.

Das Gewichtsverhältnis der Elemente Chrom und Mangan bei dem erfindungsgemäßen Trägerkatalysator beträgt etwa 5 : 1 bis 1 : 5, vorzugsweise wie 10 : 9 bis 1 : 2.

Die vorliegende Erfindung betrifft weiterhin ein Verfahren zur Herstellung eines Trägerkatalysators, das dadurch gekennzeichnet ist, daß man zunächst auf den Katalysatorträger Verbindungen des Chroms und Mangans aufbringt, anschließend den so beaufschlagten Katalysatorträger auf höhere Temperatur erhitzt, dann mit einer Rhodiumlösung tränkt, trocknet, mit einer Alkalilösung behandelt und gegebenenfalls auf den so behandelten Katalysatorträger Schwefelverbindungen aufbringt.

Das Aufbringen des Chroms und Mangans auf den Katalysatorträger kann z. B. erfolgen durch gemeinsames Ausfällen eines Mangan-Chrom-Hydroxidgemisches aus einer Chrom- und Mangansalz-Lösung mit Alkalilauge oder Ammoniak und anschließendes Auswaschen der löslichen Anteile mit Wasser. Als Chrom und Mangansalze kommen insbesondere die Sulfate, Chloride, Acetate und/oder Nitrate der genannten Elemente in Betracht. Die Abscheidung des Chroms und Mangans auf den Katalysatorträger kann auch als Ammonium-manganchromat oder Ammonium-alkali-mangan-chromat aus einer Lösung von Mangan(II)-Salzen und Ammoniumbichromat mittels Ammoniak und/oder basischen Alkaliverbindungen erfolgen. Besonders gleichmäßige und fest haftende Abscheidungen erhält man, wenn die Basenzugabe langsam und gleichmäßig unter Vermeidung größerer Konzentrationsunterschiede erfolgt. In einer bevorzugten Ausführungsform wird deshalb die Fällung mittels Harnstoff unter hydrolysierenden Bedingungen vorgenommen, wodurch die obengenannten Bedingungen besonders gut gewährleistet sind.

Nach dem Aufbringen der Chrom- und Manganverbindungen auf den Katalysatorträger wird der so beaufschlagte Katalysatorträger sulfatfrei gewaschen, bevor er auf höhere Temperaturen (ca. 200 bis 450 °C, vorzugsweise 250 bis 350 °C) erhitzt wird. Die Temperung des mit Chrom- und Manganverbindungen beaufschlagten Katalysatorträgers beträgt etwa 0,5 bis 3 Stunden, bevorzugt 1 bis 2 Stunden.

Nach der Temperung des mit Chrom und Mangan beaufschlagten Katalysatorträgers wird das Rhodium nach bekannten Methoden aufgebracht. Dies kann in der Weise erfolgen, daß man aus einer wäßrigen Rhodiumsalzlösung, z. B. einer Rhodiumtrichlorid-, Rhodiumnitrat- oder Rhodiumacetatlösung, das Rhodium durch Fällung mit Natronlauge abscheidet. Es ist auch möglich, den mit einer Mangan- und einer Chromverbindung beaufschlagten Katalysatorträger zunächst mit einer Rhodiumsalzlösung zu tränken und danach den so getränkten Katalysatorträger mit Natronlauge zu behandeln. In der gleichen Reihenfolge, d. h. zuerst Rhodiumsalzlösung und dann Alkalilösung, lassen sich auch die beiden

2

Lösungen in einer beheizten Dragiertrommel auf den Katalysatorträger aufsprühen.

Zur Nachbehandlung des rhodiumhaltigen Trägerkatalysators eignen sich z. B. wäßrige Lösungen anorganischer und/oder organischer Alkaliverbindungen, wie die Oxide, Hydroxide und/oder Alkoholate der Alkalimetalle sowie die Salze solcher Säuren, die weder selbst noch in Form ihrer Reaktionsprodukte als Hydrierkatalysator-Gifte im Sinne der üblichen Formulierung (z. B. gemäß Zymalkowski : « Katalytische Hydrierung », (1965), Seite 36 ; Houben-Weyl (1955), 4/2, Seite 257) gelten, also insbesondere solche, die frei von N, P, As, Sb, Se, Te, Cl, Br und J sind, wie die Carbonate, Bicarbonate, Acetate und/oder die Salze anderer niederer Carbonsäuren. Zum Beispiel seien als Alkaliverbindungen genannt : Lithiumhydroxid, Natriumhydroxid, Natriumcarbonat, Natriumhydrogencarbonat, Natriummethylat, Natriumethylat, Natriumacetat, Kaliumhydroxid, Kaliumcarbonat, Kaliummethylat und/oder Rubidiumhydroxid.

Die Konzentration der Alkaliverbindungen in der verwendeten Alkalilösung beträgt im allgemeinen etwa 0,02- bis 5-n, vorzugsweise 0,02- bis 2-n, insbesondere 0,01- bis 1-n.

In einer bevorzugten Ausführungsform werden auf den rhodiumhaltigen Trägerkatalysator zusätzlich Schwefelverbindungen aufgebracht. Beispielsweise seien als geeignete Schwefelverbindung genannt : die Sulfate, Sulfite, Thiosulfate und Rhodanide der Alkalimetalle, bevorzugt $K_2SO_4$, $Na_2SO_4$, $Na_2SO_3$, $Na_2S_2O_3$, KSCN und NaSCN. Diese Salze können sowohl einzeln als auch im Gemisch untereinander eingesetzt werden. Sie können aber auch zusammen mit den Alkaliverbindungen in Wasser gelöst werden und so im Gemisch auf den Trägerkatalysator aufgebracht werden.

Um die schwefelhaltigen Verbindungen auf den Katalysator aufzubringen, löst man die genannten Salze in Wasser und tränkt·oder besprüht mit dieser Lösung den Katalysator, der bereits das Rhodium, sowie Verbindungen des Chroms, des Mangans sowie der Alkalimetalle enthält.

Die genannten Schwefelverbindungen werden in einer solchen Menge auf den Trägerkatalysator aufgebracht, daß dieser einen Schwefelgehalt von etwa 0,05 bis 3 Gew.-%, bevorzugt 0,1 bis 1,6 Gew.-%, bezogen auf das Trägermaterial aufweist.

Die Trocknung des Katalysators kann bei Temperaturen im Bereich von etwa 20 bis 150 °C, bevorzugt von 50 bis 120 °C, vorgenommen werden.

Der erfindungsgemäße Katalysator kann nach dem Trocknen direkt für eine Dehydrierungsreaktion eingesetzt werden, vorteilhafter ist es jedoch, ihn vor der Verwendung mit Wasserstoff bei 120 bis 450 °C, vorzugsweise bei 350 bis 420 °C, etwa 30 bis 80 Stunden lang zu behandeln.

Der erfindungsgemäß hergestellte Katalysator eignet sich besonders zur Herstellung von Hydroxydiphenyl durch katalytische Dehydrierung von Verbindungen und/oder Verbindungsgemischen, die aus ganz und/oder teilweise hydriertem Hydroxydiphenyl bestehen.

Gegenstand der vorliegenden Erfindung ist deshalb weiterhin die Verwendung des auf die oben beschriebene Weise erhaltenen Katalysators zur Herstellung von Hydroxydiphenyl durch katalytische Dehydrierung von Verbindungen und/oder Verbindungsgemischen, die aus ganz und/oder teilweise hydriertem Hydroxydiphenyl bestehen.

Geeignete Ausgangsprodukte für die erfindungsgemäße Verwendung des Katalysators zur Herstellung von Hydroxydiphenyl sind beispielsweise : 2-Cyclohexylidencyclohexanon, 2-Cyclohexenylcyclohexanon, 2-Cyclohexylcyclohexanon, 2-Cyclohexylcyclohexanol, 2-Cyclohexylphenol, 3-Cyclohexylphenol, 4-Cyclohexylphenol, 2-Phenylcyclohexanon und 2-Phenylcyclohexanol.

Die genannten Verbindungen sind leicht zugänglich. So erhält man beispielsweise 2-Cyclohexylidencyclohexanon und 2-Cyclohexenylcyclohexanon durch Kondensieren von Cyclohexanon in Gegenwart saurer oder basischer Katalysatoren nach bekannten Methoden. Diese beiden Verbindungen entstehen außerdem neben 2-Cyclohexylcyclohexanon, 2-Cyclohexylcyclohexanol u. a. als Nebenprodukte bei der katalytischen Cyclohexanol-Dehydrierung. Sie können aus dem Dehydriergemisch leicht destillativ abgetrennt und als Gemisch für die 2-Hydroxydiphenyl-Herstellung verwandt werden.

Cyclohexylphenol gewinnt man nach bekannten Methoden durch katalytische Phenolalkylierung ; 2-Cyclohexylphenol tritt außerdem auch neben 2-Phenylcyclohexanon und 2-Phenylcyclohexanol, 2-Cyclohexylcyclohexanol und 2-Cyclohexylcyclohexanon als Nebenprodukt bei der 2-Hydroxydiphenyl-Synthese auf. Die katalytische Dehydrierung der obengenannten Verbindungen bzw. Verbindungsgemische wird im allgemeinen so durchgeführt das man die Verbindung bzw. Verbindungsgemische dampfförmig bei Temperaturen von etwa 300 bis etwa 400 °C, insbesondere von etwa 320 bis etwa 390 °C unter Normaldruck oder vermindertem Druck über den Katalysator leitet.

Das nach dem erfindungsgemäßen Verfahren hergestellte Hydroxydiphenyl wird beispielsweise als Konservierungsmittel für Citrusfrüchte oder als Carrier für das Färben von Dispersionsfarbstoffen verwendet (vgl. DE-C-20 49 809).

Beispiel 1

a) Herstellung des Katalysators

100 g kugelförmiges $\gamma$-$Al_2O_3$ (Durchmesser : 2 bis 5 mm) mit einer spezifischen Oberfläche von 350 $m^2$/g wurden in einem Rundkolben vorgelegt und mit einer Lösung von 8,3 g $MnSO_4 \cdot H_2O$, 6,2 g $(NH_4)_2Cr_2O_7$ und 45 g Harnstoff in 72 ml Wasser versetzt. Unter drehender Bewegung wurde der Kolben eine Stunde auf 85 °C gehalten, die nicht aufgenommene Flüssigkeit abfiltriert und der Katalysatorträger sulfatfrei gewaschen und dann 25 Stunden bei 110 °C unter Wasserstrahlvakuum getrocknet. Anschließend wurde der so behandelte Katalysatorträger 30 Minuten bei 300 °C getempert. Der so mit Chrom und Mangan beaufschlagte Katalysatorträger wurde nun in einem Rundkolben mit einer Lösung von 2,74 g Rhodiumtrichlorid in 30 ml Wasser gleichmäßig getränkt. Die feuchten Katalysatorpellets wurden bei 100 °C im Wasserstrahlvakuum getrocknet und danach mit einer Lösung von 2,92 g Natriumhydroxid in 30 ml Wasser erneut getränkt. Danach wurden die Katalysatorpellets 43 Stunden lang bei 100 °C im Wasserstrahlvakuum getrocknet und anschließend 66 Stunden lang bei 400 °C in einem Wasserstrom von 10 l/h getempert.

b) Verwendung des Katalysators

30 ml (25,6 g) des rhodiumhaltigen Trägerkatalysators wurden in einem senkrecht stehenden, elektrisch beheizten Glasrohr von 72 cm Länge und 17 mm Innendurchmesser auf 330 bis 350 °C erwärmt. Unter Benutzung einer geeichten Dosiervorrichtung wurden stündlich 6 g eines Gemisches aus 2-Cyclohexenylcyclohexanon und 2-Cyclohexylidencyclohexanon zusammen mit 10 l Wasserstoff in das Reaktionsrohr geleitet. Im oberen Teil des Rohres, in dem sich nur Füllkörper befanden, erfolgte die Verdampfung des flüssigen Ausgangsgemisches.

Während der ersten 48 Betriebsstunden wurde der Katalysator auf 340 °C gehalten. Dabei bildete sich ein Reaktionsprodukt das folgende Zusammensetzung aufwies :

| | |
|---|---|
| 2-Hydroxydiphenyl | 88 % |
| Diphenylenoxid | 5,7 % |
| Diphenyl | 3,3 % |
| Phenol | 0,4 % |

Nach dieser Anlaufzeit wurde eine Temperatur für den Katalysator von 325 bis 330 °C eingestellt. Nach 1278, 2017, 3179 und 5770 Betriebsstunden wurde ein Reaktionsgemisch folgender Zusammensetzung erhalten :

| | 1278 h | 2017 h | 3179 h | 5770 h |
|---|---|---|---|---|
| 2-Hydroxydiphenyl | 85,2 % | 85,1 % | 85,4 % | 86,6 % |
| 2-Cyclohexylphenol | 0,8 % | 1,1 % | 1,1 % | 1,6 % |
| Diphenylenoxid | 8,7 % | 8,6 % | 8,7 % | 8,1 % |
| Diphenyl | 3,1 % | 3,0 % | 2,8 % | 2,1 % |
| Phenol | 0,3 % | 0,3 % | 0,3 % | 0,4 % |

Beispiel 2

Die Arbeitsweise des Beispiels 1 wurde wiederholt, mit der Ausnahme, daß nur 0,5 Gew.-% Rhodium, bezogen auf das Trägermaterial, auf den Katalysator aufgebracht wurde und die Reaktionstemperatur bei der Dehydrierung auf 350 °C eingestellt wurde. Innerhalb von 191 Stunden wurden 1 191 g des in Beispiel 1 beschriebenen Ausgangsgemisches eingesetzt und daraus 1 128 g Reaktionsprodukt erhalten. Das Reaktionsprodukt hatte in Abhängigkeit von der Einsatzdauer des Katalysators folgende Zusammensetzung :

| | 89 h | 126 h | 191 h |
|---|---|---|---|
| 2-Hydroxydiphenyl | 84,7 % | 84,8 % | 84,4 % |
| 2-Cyclohexylphenol | - | - | - |
| Diphenyl | 5,3 % | 5,1 % | 5,1 % |
| Diphenylenoxid | 6,1 % | 6,5 % | 6,8 % |
| Phenol | 1,2 % | 1,1 % | 1,0 % |

Beispiel 3 (Vergleichsbeispiel)

Entsprechend dem Beispiel 1a) wurden auf 100 g kugelförmiges $\gamma$-$Al_2O_3$ (Durchmesser : 2 bis 5 mm) mit einer spezifischen Oberfläche von 350 m²/g entsprechend der DE-A-22 11 721 0,5 Gew.-% Rhodium, bezogen auf das Trägermaterial, aufgebracht ohne weiteren Zusatz der in Beispiel 1 genannten Metalle.

4

30 ml (27 g) dieses Katalysators wurden entsprechend den Bedingungen des Beispiels 2 zur Dehydrierung eingesetzt. Das Reaktionsprodukt, das im Verlauf von 60 Stunden erhalten wurde, enthielt weniger als 5 Gew.-% des gewünschten 2-Hydroxydiphenyls.

Dieses Vergleichsbeispiel verdeutlicht, daß ein Trägerkatalysator der nur Rhodium enthält für die technische Herstellung von 2-Hydroxydiphenyl ungeeignet ist.

### Beispiel 4

Unter Verwendung von 30 ml (24 g) des gemäß Beispiel 1 hergestellten Katalysators wurde die Dehydrierungsreaktion ohne Trägergas (d. h. ohne Wasserstoff) durchgeführt. Dabei wurde die Temperatur im Reaktionsofen zunächst auf 300 °C und der Durchsatz des Ausgangsgemisches (2-Cyclohexenylcyclohexanon und 2-Cyclohexylidencyclohexanon) auf 6,2 g/h eingestellt. Im Verlauf dieses Dehydrierungsversuches wurde die Temperatur des Reaktionsofens stufenweise bis 385 °C und der Durchsatz an Ausgangsgemisch auf 26 g/h erhöht. Die folgende Tabelle gibt den Gehalt an 2-Hydroxydiphenyl und an 2-Cyclohexylphenol im Reaktionsprodukt in Abhängigkeit von den Betriebsstunden des Katalysators an. Das in der letzten Spalte aufgeführte 2-Cyclohexylphenol kann wieder in die Reaktion als Ausgangsprodukt eingesetzt werden.

| Betriebs-stunden | Temp. (°C) | Durchsatz g/h | im Reaktionsprodukt (%) | |
|---|---|---|---|---|
| | | | 2-Hydroxy-diphenyl | 2-Cyclohexyl-phenol |
| 72 | 300 | 6,2 | 60,1 | 23,4 |
| 210 | 330 | 6,2 | 83,6 | 2,5 |
| 362 | 350 | 12,7 | 86,7 | 1,4 |
| 422 | 370 | 26,1 | 85,8 | 4,7 |
| 433 | 380 | 26,5 | 87,1 | 2,4 |
| 527 | 380 | 26,1 | 86,1 | 2,9 |
| 635 | 385 | 25,9 | 86,0 | 3,8 |

### Beispiel 5

27 g des gemäß Beispiel 1 hergestellten Katalysators wurden zusätzlich mit einer Lösung von 0,81 g $K_2SO_4$ in 10 ml Wasser getränkt und danach 20 Stunden bei 120 °C getrocknet. 30 ml (25,5 g) des so hergestellten Katalysators wurden unter Benutzung des in Beispiel 1 beschriebenen Reaktionsrohres im Wasserstoffstrom (10 l/h) auf 400 °C erwärmt und 68 Stunden bei dieser Temperatur gehalten. Dann wurde die Ofentemperatur auf 350 °C gesenkt und die Dehydrierungsreaktion ohne Trägergas durchgeführt. Dabei wurden von dem Isomerengemisch 2-Cyclohexenylcyclohexanon und 2-Cyclohexylidencyclohexanon 11,7 g/h eingesetzt. Im Verlauf von 358 Stunden wurden 3 972 g Reaktionsprodukt gewonnen, das 92,3 % o-Phenylphenol und 0,5 % Cyclohexylphenol enthielt.

### Beispiel 6

15 g des gemäß Beispiel 1 hergestellten Katalysators wurden zusätzlich mit einer Lösung von 0,15 g KSCN in 6 ml Wasser getränkt und anschließend 94 Stunden bei 120 °C getrocknet. 12,3 g des so erhaltenen Katalysators wurden im Wasserstoffstrom (10 l/h) auf 400 °C erwärmt und 62 Stunden bei dieser Temperatur gehalten.

Über den so reduzierten Katalysator wurden bei 350 bis 375 °C etwa 6 g eines Isomerengemisches aus 2-Cyclohexenylcyclohexanon und 2-Cyclohexylidencyclohexanon geleitet. Dabei wurden im Verlauf von 245 Stunden 1 387 g eines Reaktionsgemisches erhalten, das 90 % o-Phenylphenol und 2,8 % 2-Cyclohexylphenol enthielt.

**Patentansprüche**

1. Trägerkatalysator, dadurch gekennzeichnet, daß er einen Gehalt an Rhodium von 0,1 bis 5 Gew.-%, einen Gehalt an Chrom und Mangan von zusammen 0,05 bis 8 Gew.-%, einen Gehalt an Alkalimetall von 0,05 bis 15 Gew.-% und gegebenenfalls einen Schwefelgehalt von 0,05 bis 3 Gew.-%, bezogen auf das Trägermaterial, aufweist.

2. Verfahren zur Herstellung des Trägerkatalysators nach Anspruch 1, dadurch gekennzeichnet, daß man zunächst auf den Katalysatorträger Verbindungen des Chroms und Mangans aufbringt, anschließend den so beaufschlagten Katalysatorträger auf höhere Temperaturen erhitzt, dann mit einer Rhodiumlösung tränkt, trocknet, mit einer Alkalilösung behandelt und gegebenenfalls auf den so behandelten Katalysatorträger Schwefelverbindungen aufbringt.

3. Verwendung des Trägerkatalysators nach Anspruch 1 zur Dehydrierung von Verbindungen und/oder Verbindungsgemischen, die aus ganz und/oder teilweise hydriertem Hydroxydiphenyl bestehen.

## Claims

1. Supported catalyst, characterized in that it has a content of rhodium of 0.1 to 5 % by weight, a content of chromium and manganese together of 0.05 to 8 % by weight, a content of alkali metal of 0.05 to 15 % by weight and, if appropriate, a sulphur content of 0.05 to 3 % by weight, based on the carrier material.

2. Process for the preparation of the supported catalyst according to Claim 1, characterized in that compounds of chromium and manganese are first applied to the catalyst support and the catalyst support thus charged is subsequently heated to, elevated temperatures and then impregnated with a rhodium solution, dried and treated with an alkali metal solution, and, if appropriate, sulphur compounds are applied to the catalyst support thus treated.

3. Use of the supported catalyst according to Claim 1 for the dehydration of compounds and/or compound mixtures consisting of completely and/or partly hydrogenated hydroxydiphenyl.

## Revendications

1. Catalyseur sur support, caractérisé en ce qu'il présente une teneur en rhodium de 0,1 à 5 % en poids, une teneur en chrome plus manganèse de 0,05 à 8 % en poids, une teneur en métal alcalin de 0,05 à 15 % en poids et, le cas échéant, une teneur en soufre de 0,05 à 3 % en poids, par rapport à la matière de support.

2. Procédé de préparation du catalyseur sur support suivant la revendication 1, caractérisé en ce qu'on dépose tout d'abord sur le support de catalyseur des composés de chrome et de manganèse, puis on chauffe le support de catalyseur ainsi chargé à des températures élevées, après quoi on l'imprègne de solution de rhodium, on le sèche, on le traite avec une solution d'alcali et on dépose éventuellement des composés du soufre sur le support de catalyseur ainsi traité.

3. Utilisation du catalyseur sur support suivant la revendication 1 pour la déshydrogénation de composés et/ou de mélanges de composés qui sont constitués d'hydroxydiphényle totalement et/ou partiellement hydrogénés.